# EUROPEAN PATENT SPECIFICATION

(11) **EP 3 654 892 B1**
(45) Date of publication and mention of the grant of the patent: **02.12.2026**
(21) Application number: 18834866.8
(22) Date of filing: 17.07.2018
(51) Int. Cl.: A61F 5/44, A61F 5/441, A61F 5/445, A61F 5/448, A61F 5/449

(54) **OSTOMY POUCHING DEVICE**
BEUTELVORRICHTUNG FÜR STOMA
DISPOSITIF POCHE D'OSTOMIE

(30) Priority: 17.07.2017 US 201762533168 P
(43) Date of publication of application: 27.05.2020
(73) Proprietor: Lin, Chih-hao, Taipei City 116 (TW); Shen, Wan-chen, Taipei City 116 (TW); Shih, Wei-ting, Taoyuan 320 (TW)
(72) Inventor: Lin, Chih-hao, Taipei City 116 (TW); Shen, Wan-chen, Taipei City 116 (TW); Shih, Wei-ting, Taoyuan 320 (TW)
(74) Representative: Hernandez, Yorck
(86) International application number: PCT/US2018/042570
(87) International publication number: WO 2019/018452

(56) References cited:
- EP-A1- 0 768 849
- WO-A1-2016/008981
- US-A- 3 828 782
- US-A- 4 826 495
- US-A- 4 826 495
- US-A- 4 938 750
- US-A- 5 591 144
- US-A- 5 591 144
- US-A- 5 690 623
- US-A- 5 785 695
- US-A1- 2008 262 449
- US-A1- 2011 092 929
- US-A1- 2011 196 323
- US-A1- 2011 196 323
- US-A1- 2012 136 324
- US-A1- 2012 232 505
- US-A1- 2012 234 485
- US-A1- 2015 305 916
- US-A1- 2015 320 585
- US-B2- 9 629 743

## Description

### FIELD OF INVENTION

The present invention is related to an ostomy pouching device.

### BACKGROUND OF THE INVENTION

An ostomy operation is often necessary as part of treatment to various medical conditions, such as surgery to remove cancerous growth, which requires the removal of part or all of a patient's bowels, such as the small or large intestine. After the surgery, if the bowels are not reattached, the waste from the bowels, such as urine and feces, are usually removed from the body through an artificial opening in the patient's abdominal wall.

Methods presently available to collect such waste often involve the use of a reservoir. A conventional method for collecting and disposing of waste calls for using an ostomy bag connected to the bowel. The bag usually must be emptied and cleaned frequently, usually every several hours. The ostomy bag is usually positioned outside of the abdomen, and often taped to the patient's skin.

There are several drawbacks associated with the conventional methods. First, the connection between the stoma and the ostomy bag are often not 100% secure. Leakage and odor are common issues faced by patients. The stoma often requires special care to prevent irritation and infection. Similarly, the skin to which the ostomy bag is adhered becomes irritated as well.

Furthermore, using and wearing an ostomy bag may create major quality of life issues. The fear of embarrassment of possible leakage and odor often lead to self-imposed social isolation, as patients limit their physical and social activities. This may lead to depression and other psychological suffering.

Accordingly, it is desirable to provide an improved device and method that overcomes drawbacks and inadequacies of known methods and devices.

US 2011/196323 A1 discloses an ostomy bag comprising an outwardly facing irrigation line attachment adapter, a removable vent housing attached to the attachment adapter, where the vent housing is attached during normal operation of the ostomy bag, where the vent housing may comprise a charcoal filter and a scent absorbing medium for reducing odors which may vent from the ostomy bag. The vent housing is attached with quick-connect connectors, and is easily removed so that a conduit may be attached to the attachment adapter, where the conduit is attached to a source of flushing liquid, such as a squeezable reservoir or faucet.

EP 0 768 849 A1 discloses a drainage bag for receiving bodily waste, the drainage bag comprises a water-impermeable inner bag enclosed therein; means defining an orifice to enable bodily waste to be received by the inner bag; the outer and inner bags being detachably secured together in the region of the orifice. A first opening is provided in a wall of the water-impermeable inner bag, said first opening being covered by a portion of gas-permeable hydrophobic foam material; and a second opening is provided in a wall of the water-impermeable outer bag, the second opening being covered by a flatus filter; the disposition of the first and second openings being such as to allow flatus gases within the inner bag to pass out through the first opening and thence through the second opening to atmosphere.

US 5 690 623 A discloses vented ostomy pouches in which a first wall is securable to a patient's peristomal skin surfaces and the obverse second wall is provided with an outlet opening for the escape of gases from the pouch. A deodorizing gas filter extends over the outlet and a third wall, or internal barrier wall, is located within the pouch and divides it into two chambers. The barrier wall operates to block the passage of solids and most liquids from the first chamber to the second but is provided with at least one vent that permits the flow of gases into the second chamber from the first. A filter-protecting panel in the second chamber directs such gases along a circuitous route as they travel from the vent to the outlet. A connection, in the form of a spot weld, joins the barrier wall to the bodyside first wall of the pouch at about the elevation of the filter to prevent the barrier wall from blocking against other surfaces and obstructing the flow of gases through the vent and filter.

US 4 826 495 A discloses a re-usable coupling means for fastening a flexible disposable ostomy bag to the abdominal wall of an ostomy patient for collecting intestinal wastes from an ostomy. The re-usable coupling means consists (a) of a retainer plate of a comparatively stiff plastic material having an opening to lie around the stoma and having a gas passage duct communicating with a deodorizing filter, and (b) a flexible, sealing adhesive member for releasable adherence of the retainer plate to the skin and indirectly secured to the rear of the retainer plate, the gas passage duct is arranged in an annular bead protruding from the front of the retainer plate and communicates via gas inlet openings with the interior of the bag and via the filter and gas outlet openings in the coupling means with the outer world. The disposable bag may be releasably affixed to the holding means by the aid of a pressure-sensitive adhesive or by the aid of ring coupling systems known per se. This coupling means is particularly well suited for use in ostomy bag systems of the kind consisting of a wc-flushable inner bag of a laminated sheet material with an outer layer of water-soluble film and a water- and gas-proof, mechanically weak inner layer; and an outer re-usable bag of a watertight, mechanically strong material.

US 3 828 782 A discloses a temporary colostomy tube for inserting through an incision in the colon and including an open ended excrement tube having an inflatible balloon at one end thereof. A retainer means secures the opposite end of the tube on the outside of the body, which prevents the tube from moving further inward in the colon. A band is positioned around the colon adjacent the balloon. The balloon is inflated to occupy the entire lumen and is slightly larger in circumference than the band. The cooperation of the band with the balloon blocks the tube from moving toward the incisions. The cooperation of the retainer means and the band with the balloon prevents the fecal stream from seeping around the balloon and tube. A disposal bag is secured to the tube on the outside of the body for receiving the fecal matter.

US 2011/092929 A1 discloses A stoma extender includes a first end for insertion into a stoma for diverting stomal effluent into the stoma extender before the effluent exits the stoma; a second end for remaining external of the stoma, for providing a discharge exit for stomal effluent; and a conduit portion coupled between the first and second ends for communicating stomal effluent through the stoma extender, wherein the length of the conduit portion is adjustable stably, to permit adaptation of the stoma extender to an individual's stoma.

US 2012/136324 A1 discloses an artificial Ostomy containment device comprising: an implantable intra-abdominal sleeve adapted to interfere with movement of a closure assembly positioned within the sleeve; and a closure assembly comprising a hollow stomal insert sized and shaped to conduct waste content from an intestinal portion in an abdominal cavity through said insert and out of a stoma, and a fixation element coupled to said stomal insert sized and positioned to interfere with said intra-abdominal sleeve.

### SUMMARY OF THE INVENTIONS

It is an object of the present invention to provide an improved ostomy pouching device.

The object is achieved by the features of independent claim 1. Further embodiments are defined in the dependent claims.

Still other objects and advantages of the invention will in part be obvious and will in part be apparent from the specification. Other features and advantages of this invention will become apparent in the following detailed description of exemplary embodiments of this invention with reference to the accompanying drawings.

### BRIEF DESCRIPTION OF THE DRAWINGS

For a fuller understanding of the invention, reference is made to the following description taken in connection with the accompanying drawing, in which:
FIG. 1 is an exploded perspective view of an ostomy pouching device in accordance with an embodiment of the invention;
FIG. 2 is a top view of the components of FIG. 1;
FIG. 3 is a side cross-sectional view of the device of FIG. 1;
FIG. 4 is a cross-sectional view of a bowel connector in accordance with an embodiment of the invention having an inflated inner tube balloon and an inflated outer tube balloon;
FIG. 5 is a cross-sectional view of the bowel connector of FIG. 4 having a deflated outer tube balloon;
FIG. 6 is a side view of an inner tube in accordance with an embodiment of the invention;
FIG. 7 is a side view of an outer tube in accordance with an embodiment of the invention;
FIG. 8 is a top view of an ostomy pouching device in accordance with an embodiment of the invention;
FIG. 9 is a side cross-sectional view of the device of FIG. 8;
FIG. 10 is an illustration of an ostomy pouching device in accordance with an embodiment of the invention implanted into a human body; and
FIG. 11 is cross-sectional view of a bowel connector in accordance with an embodiment of the invention.

### DETAILED DESCRIPTION OF THE PREFERRED EMBODIMENTS

The invention generally is directed to an ostomy pouching device 1 comprising an outer container referred to herein generally as "container," and preferably an inner waste collection pouch referred to herein generally as "inner bag," for placement within the container. The container may be located either inside or outside of the body. The container is implanted between the skin and the muscle, preferably the abdominal muscle. The term "container" is used herein generally to refer to any receptacle capable of housing a pouch therein, by way of non-limiting example, a shell having a defined shape, or a pouch that is flat when empty and expands as it is filled.

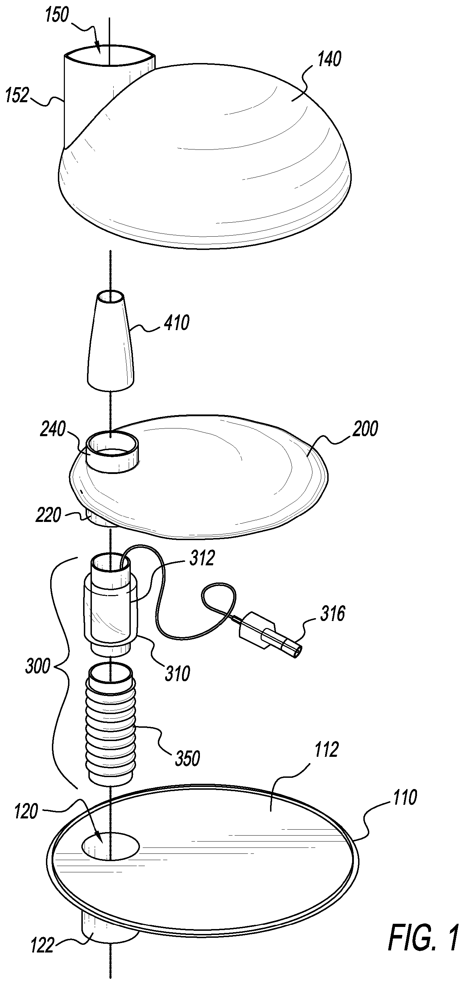

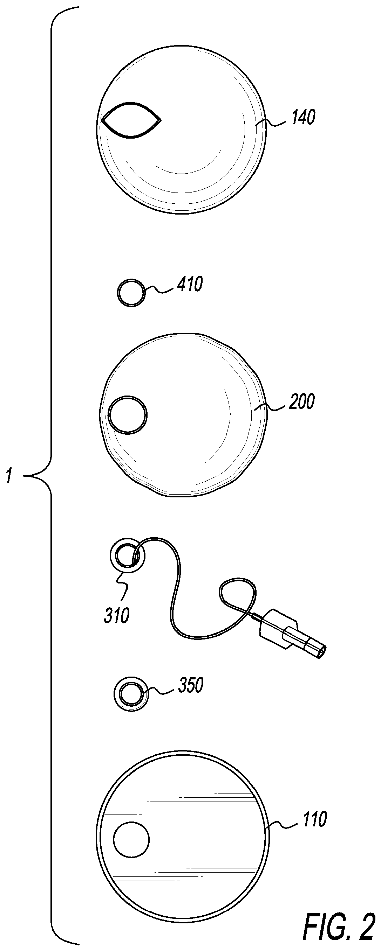

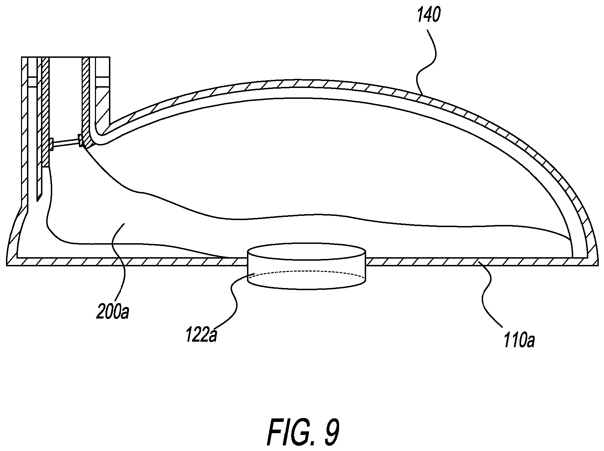

Reference is made to FIGS. 1-11, wherein certain embodiments of the invention are illustrated. In the embodiments illustrated, the device 1 includes a container 100 having a base 110, an upper member 140, and a container cavity 160. Base 110 has a base wall 112, which is generally flat and smooth. Base wall 112 includes a base aperture 120, and preferably a base tunnel 122 defining base aperture 120, which is designed to be connected to the bowel of the user, such as a patient. Base wall 112 includes an inner wall surface which faces the upper member 140 when base 110 and upper member 140 are assembled. Base wall 112 further includes an outer wall surface which faces the user's bowels when implanted in the user's body. Whereas the embodiments of base 110, 110a illustrated are shown as having a flat surface, it is to be understood that other shapes are contemplated. For example, base 100 may have a curved or wavy shape without deviating from the scope of the invention.

Upper member 140 preferably has a bulbous shape, for example, a dome shape as illustrated. An upper aperture 150 is defined by an upper tunnel 152, through which the contents of container 100, more preferably the contents of an inner bag 200 located within container 100, may be removed. Preferably, an air exit aperture 154 is provided through which air or other gas within container cavity 160 can exit container 100. In the embodiments shown, air exit aperture 154 is located proximate or within upper tunnel 152, and is open at all times, permitting air from container cavity 160 to exit. Air exit aperture 154 may include a valve or other mechanism to allow manual release of air from container cavity 160.

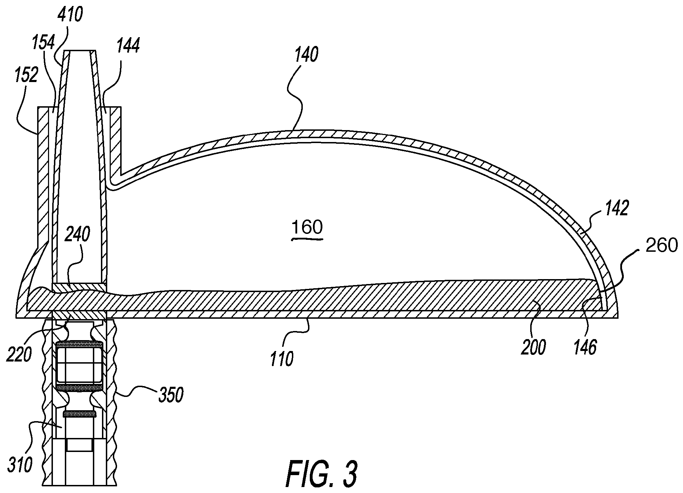

Upper member 140 may also include a gas tunnel 142 through which gas may flow from within container 100 and/or inner bag 200 to a gas exit 144. Gas exit 144 may be located proximate or incorporated into upper tunnel 152. In the embodiment shown in FIG. 3, gas exit 144 is integrated into upper tunnel 152. Gas tunnel 142 may be integral to upper member 140, built into the wall of upper member 140, or it may be a separate element, such as a tube. Furthermore, whereas the illustrations show a single gas tunnel 142, a plurality of gas tunnels 142 may be provided. The gas tunnel entrance 146 is preferably located above base aperture 120 and base tunnel 122, which connects to the bowel, and more preferably above upper aperture 150 and upper tunnel 152, when the user having device 1 implanted or otherwise connected to his body is standing. Since heavier liquids and solids would fall downward due to gravity, and the lighter gas would rise up, the gas preferably separates from the liquids and solids, and enters gas tunnel 142. Gas tunnel entrance 146 may include a valve to restrict and prevent solid and/or liquid from entering gas tunnel 142. Gas exit 144 may alternatively connect to a separate container. Gas exit 144 may always stay open or include a valve or other mechanism to selectively release gas from within gas tunnel 142. Preferably, gas exit 144 may be opened independently of external tube 410 or upper aperture 150.

Container 100 may be manufactured from a variety of material. A preferred material is silicone. Other non-limiting examples include nylon, polypropylene, and titanium. Preferably, at least for a device 1 being implanted in a user's body, container 100 is made of a material stable as an implant. Furthermore, container 100 is preferably flexible enough to bend inward and squeeze an inner bag 200 therein when pressured is applied inward onto container 100, for example, when emptying inner bag 200.

In accordance with a preferred embodiment, an inner bag 200 is located within container 100. Preferably, inner bag 200 is collapsible, expanding as contents enter it. Preferably, inner bag is produced from vinyl plastics, more preferably PVC (polyvinyl chloride). Other materials are contemplated without deviating from the scope of the invention. As inner bag 200 is filled, for example, with waste such as urine and feces, the air within container cavity 160 is displaced, and exits container 100 through air exit aperture 154. In the embodiments shown, inner bag 200 includes at least two apertures, each connected to a connecting element. For example, a first aperture to connect to a bowel connection 220 arranged to connect to a bowel connector 300 to connect to the user's bowel through which contents enter inner bag 200 from the user's bowels, and a second aperture to connect to an external connection 240 arranged to connect to a tube through which the contents of inner bag 200 are removed. A gas exit 260 may also be provided, through which gas exits inner bag 200. Preferably, gas exit 260 is connected to gas tunnel entrance 146 of container 100, so that gas from within inner bag 200 exits inner bag 200 into gas tunnel 142 and exits container 100 through gas exit 144.

Alternatively, inner bag 200 may be made of a water resistant but gas permeable material, or include a water resistant but gas permeable portion, permitting gas to exit therethrough while keeping the liquid and solid inside. The gas may exit inner bag 200 into container cavity 160 and exit through air exit aperture 154. This gas permeable portion may be provided in lieu of an opening to connect directly to the gas tunnel 142, or in combination thereof. Gas tunnel entrance 146, gas exit 144, gas tunnel 142, and/or upper aperture 150 may include a filter or smell absorbing or filtering component.

Gas often occupies a substantial volume of an ostomy pouch, precious volume that may otherwise be occupied by solid or liquid waste. For example, the average small intestine passes between 500 to 1000ml of fluid a day, and between 500 to 1800ml of gas. By permitting the removal of gas from inner bag 200 independently of solid or liquid waste, the device 1 may prolong the use of device 1 before requiring emptying thereof.

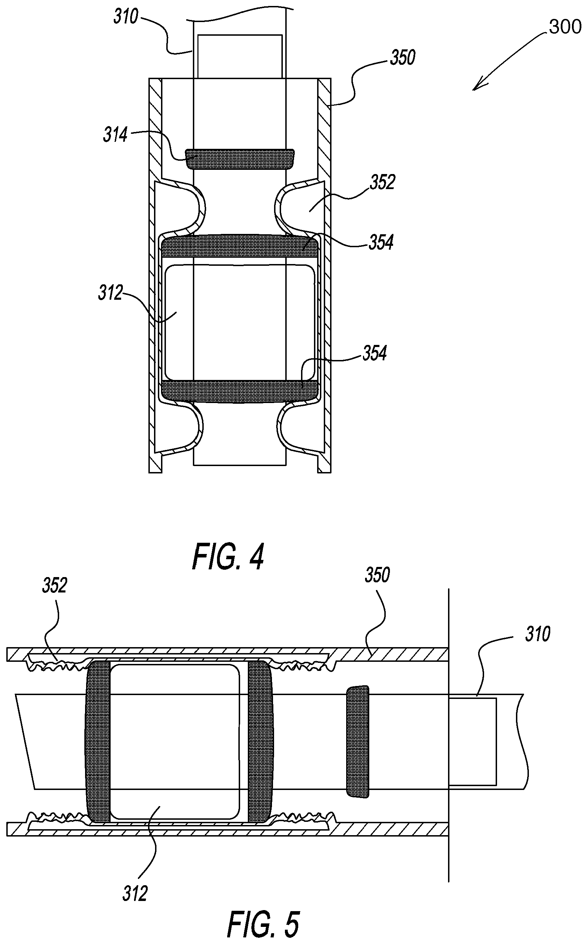

The device 1 preferably includes a bowel connector 300 to connect inner bag 200 and container 100 to the user's bowel. In the embodiments shown, bowel connector 300 extends through base tunnel 122 of container 100. Reference is made to FIGS. 4-7 illustrating a preferred embodiment of bowel connector 300. Bowel connector 300 includes an inner tube 310 and outer tube 350. An outer member may be provided to wrap around outer tube 350 to create an anti-adhesion membrane. Bowel connector 300 may be integrated with bowel connection 220 and base tunnel 122, preferably so that there is no gap between the bowel and base tunnel 122. For example, inner tube 310 may be integrated with inner bag 200, and outer tube 350 may be integrated with base 110, preferably at base tunnel 122. Alternatively, as shown in the figures, inner tube 310 may be a separate piece that connects to bowel connection 220 of inner bag 200, preferably at bowel connection 220. Likewise, outer tube 350 may be a separate piece that connects to base 110 through base tunnel 122 or connects to base tunnel 122.

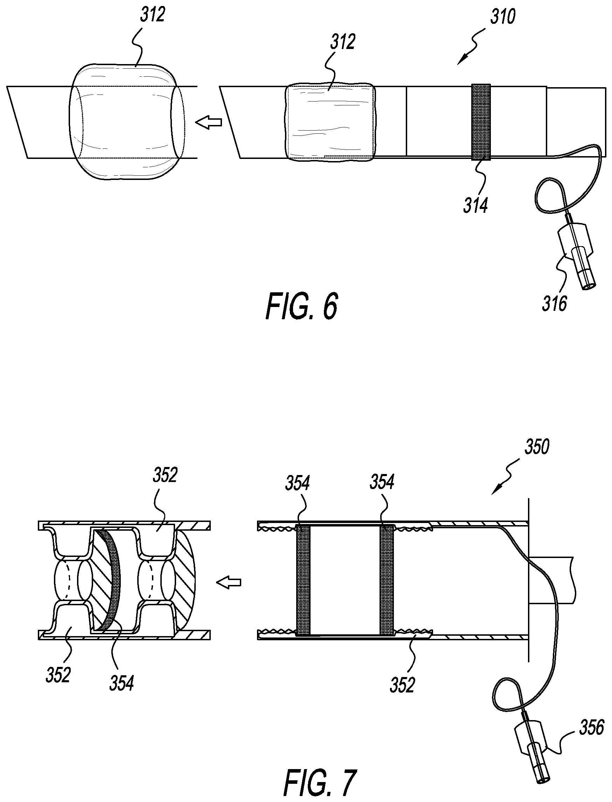

Reference is made to the embodiment of bowel connector 300 shown in FIGS. 4-7. When in use, the end of the user's bowel is inserted into outer tube 350 and over inner tube 310, being sandwiched therebetween. Both inner tube 310 and outer tube 350 include one or more balloons for securing the bowel in place, as illustrated in FIG. 4. Inner tube 310 includes a cylindrical, donut-shaped inner tube balloon 312 on the exterior thereof as shown in FIG. 6, such that the outer diameter increases when the inner tube balloon 312 is inflated, thus exerting pressure on the bowel from the inside. Outer tube 350 includes two outer tube balloons 352 on the inside thereof, such that the inner diameter decreases when the outer tube balloons 352 are inflated as shown in FIG. 7. Thus, when the bowel is positioned between outer tube 350 and inner tube 310, the inner tube balloon 312 pushes outward onto the bowel and outer tube balloons 352 exert pressure inward onto the bowel. In the embodiment shown, tube balloons 352 are on either end of inner tube balloon 312 to prevent the displacement of inner tube 310. FIG. 4 illustrates bowel connector 300 having outer tube balloons 352 and inner tube balloon 312 inflated. In FIG. 5, outer tube balloons 352 are deflated, and thus inner tube 310 may be displaced. A pump 316, 356 may be provided for inflating inner tube balloon 312 and/or outer tube balloon 352 through a tube. The pump 316, 356 preferably extends through upper aperture 150 to the exterior of container 100 to permit the user to inflate the balloons. Preferably, pump 316, 356 may be used to deflate the balloons to facilitate removal of bowel connector 300 from the bowel.

Inner tube 310 and outer tube 350 shown also include biocompatible surfaces 314, 354 to permit human tissue to grow and bond thereto. Non-limiting examples include Polyethylene terephthalate and polytetrafluoroethylene (PTFE/Teflon). Biocompatible surface 314 of inner tube 310 is located on the outer surface of inner tube 310 to contact the inner surface of the bowel. Likewise, biocompatible surface 354 of outer tube 350 is located on the inner surface of outer tube 350 to contact the outer surface of the bowel. The biocompatible surface 314, 354 may contact the serosa and the sero-muscular edge of the ileostomy. During the healing process, the serosa may grow to adhere to the biocompatible surface 314, 354.

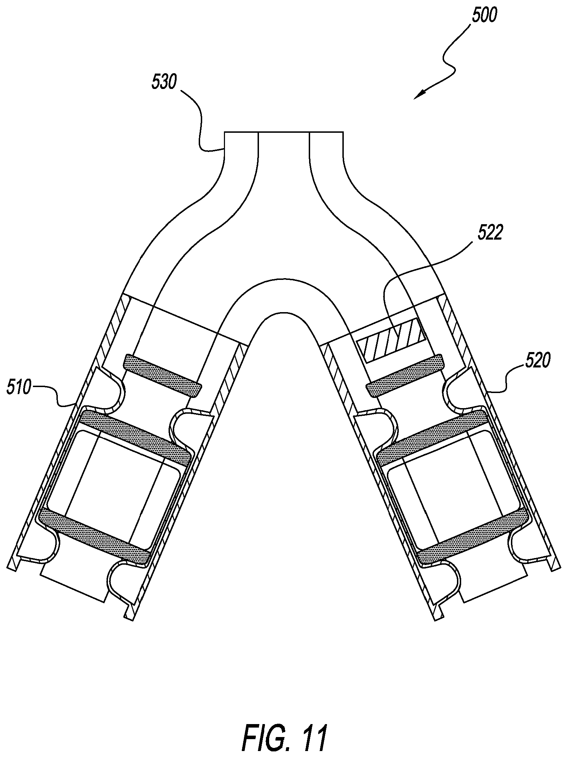

An alternate embodiment of a bowel connector 500 is illustrated in FIG. 11, wherein a Y-shape connector has three legs 510, 520, 530, each having an opening. First leg 510 connects to the upstream end of the user's bowel, second leg 520 connects to the downstream end of the bowel, and third leg 530 connects to the inner bag 200 and/or container 100. First leg 510 preferably includes an inner tube and outer tube, with a securing mechanism such as balloons as discussed with respect to the embodiment above. Second leg 520 may also include a similar inner tube and outer tube assembly. Preferably, second leg 520 further includes a stopper 522 to prevent content from the upstream end of the bowel entering first leg 510 from entering the downstream end of the bowel connected to second leg 520. More preferably, the legs include a control mechanism to open or close the opening of the respective leg. For example, when the device is implanted, the second leg 520's opening is closed, and the openings of first leg 510 and third leg 530 are open. Thus, content from the bowel enters the inner bag or container. After the user has received treatment for a few months, for example 3 to 6 months, or any other situation in which a temporary reconnection of the bowel is wanted, the opening of third leg 530 may be closed and the opening of second leg 520 is opened. Thus, content may flow from the upstream end of the bowel to the downstream end of the bowel. This reconnection may be desired to observe symptoms/issues of reconnection procedures, and allow various diagnostics, such as scopes, videos, etc., which may reduce the risk of issues after reconnecting the intestines.

An embodiment of device 1 also includes an external connector comprising external tube 410 and upper tunnel 152. External tube 410 connects to external connection 240 of inner bag 200, and extends through the inside of upper tunnel 152. The contents of inner bag 200 are removed through external tube 410. A cover 170 covers upper external tube 410, such that when cover 170 is removed, the contents of inner bag 200 or container 100 may be removed, preferably by applying pressure onto container 100. In accordance with an embodiment of the invention, cover 170 also covers upper tunnel 152 partially or entirely. In use, the user may remove cover 170 and press onto container 100, preferable displacing upper member 140 toward base 110, thus applying pressure onto the contents of container 100 and thus inner bag 200, pushing the contents through external tube 410. In accordance with an embodiment of the invention, cover 170 also covers gas exit 260.

The device 1 may include one or more sensors to detect the fullness, pressure, and/or weight of the inner bag 200 or container 100. A pressure sensor may sense the pressure in the gas tunnel 142. Another pressure sensor may measure the pressure within inner bag 200. Yet another sensor may measure the weight and fullness of inner bag 200. If container 100 receives the content from the bowel without an inner bag 200, one or more sensors may measure the pressure, weight, and/or fullness of container 100. One or more valves may be provided for the air exit aperture 154 and/or gas exit 144, and automatically released to relieve pressure in the container 100 or gas tunnel 142. Alternatively, the user may manually release the air or gas.

Preferably, inner bag 200 has a smaller volume than container 100. In accordance with an embodiment of the invention, container 100 has a length of between 5 to 20cm, more preferably approximately 10cm, the volume of container 100 is between 100 to 500ml, more preferably between 200-300ml. The thickness of container 100 may vary, for example, depending on the size of the user, whether it is implanted or attached externally. In accordance with a preferred embodiment, container 100 has a thickness of 15cm or less, more preferably between 2 to 6cm, when inner bag 200 is full.

Whereas the illustrated embodiments of container 100 have a generally round shape, it is to be understood that container 100 may have another shape without deviating from the scope of the invention. Preferably, container 100 lacks sharp or hard edges.

An embodiment of the invention provides a container into which content from the bowel enters the container without an inner bag therein. Bowel connector 300 connects directly to the base of the container. Preferably, the container expands as it is filled, and does not have substantive air that is displaced by the content. Alternatively, the air within the container may be displaced via the gas exit.

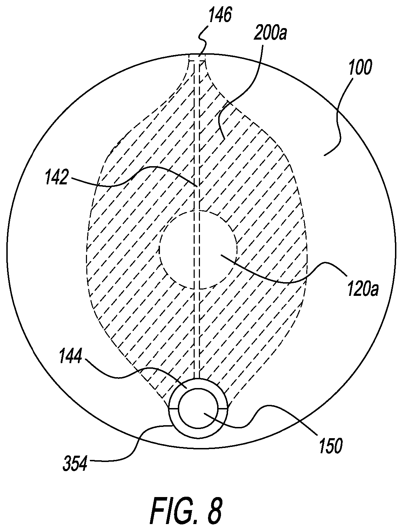

Preferably, upper aperture 150 is aligned with or proximate base aperture 120 as shown in the embodiment illustrated in FIGS. 1-3. Such an arrangement may facilitate the removal/replacement of inner bag 200 or bowel connector 300, 500, troubleshooting issues with bowel connector 300, 500, adjusting the stopper 522, etc. In an alternative embodiment illustrated in FIGS. 8-9, upper aperture 150 is located lower than base tunnel 122a of base 110a when the patient using the device is standing. Likewise, the corresponding inner bag 200a has a bowel connection aligned with base tunnel 122a, and thus higher than upper aperture 150. Providing an upper aperture 150 lower than base tunnel 122a through which waste enters inner bag 200a may facilitate collecting waste in inner bag 200a with the assistance of gravity. It may also facilitate the removal of the waste from inner bag 200a.

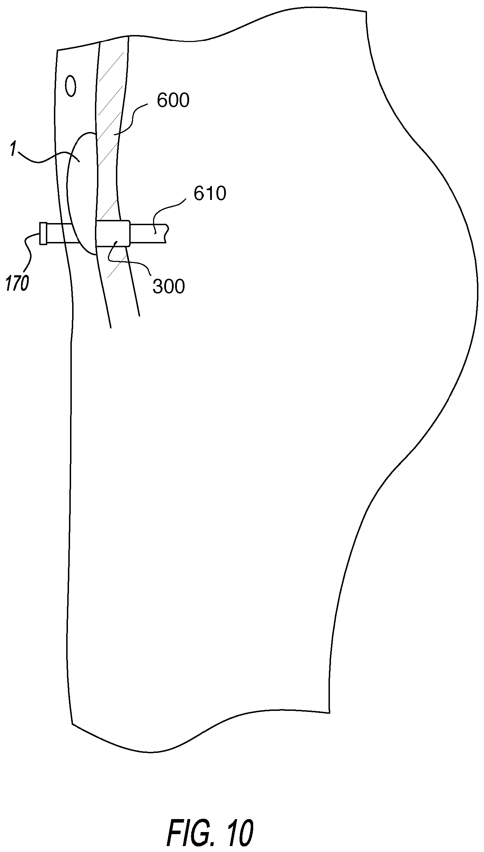

Device 1 may be implanted inside the user's body, or it may be utilized externally and maintained outside of the body. In accordance with a preferred embodiment illustrated in FIG. 10, device 1 is implanted between the user's skin and abdominal muscle 600, and the outer wall surface of base 110 contacts the user's abdominal muscle. Bowel connector 300 extending into the body connects to the bowel 610 to receive waste therefrom.

Other alterations may be made without deviating from the scope of the invention. Accordingly, the device, the components thereof, and method of use, etc. may be varied as a matter of application specific design choice without deviating from the scope of the invention. For example, different mechanisms for securing the bowel to the container 100 and/or inner bag 200, positions of the upper aperture, base aperture, gas tunnel, gas exit, gas tunnel entrance, air exhaust etc. are contemplated. Whereas the illustrated embodiments provide the gas exit integrated with the upper aperture, a separate gas exit may be provided, for example, higher up on container 100 when the user is standing, with or without a gas tunnel. It is the intention, therefore, to be limited only as indicated by the scope of the claims appended hereto.

It is also to be understood that the following claims are intended to cover all of the generic and specific features of the invention herein described and all statements of the scope of the invention which, as a matter of language, might be said to fall there between.

## Claims

1. An implantable ostomy pouching device (1) comprising:
a container (100) having a container cavity (160), the container (100) configured to be entirely implanted between the skin and the abdominal muscles of a user, the container (100) defined by a proximal base (110) with a base wall (112) and a distal member (140), the proximal base (110) and the distal member (140) defining the container cavity (160) when assembled, the base wall (112) having a base tunnel (122) defining a base aperture (120) designed to be connected to the bowel of the user, and the distal member (140) having a distal tunnel (152) defining a distal aperture (150), the distal tunnel (152) configured to traverse through subcutaneous tissues under the skin of the user, to connect the container cavity (160) to the exterior of the skin;
an inner bag (200) located inside the container (100), the inner bag (200) having a bag bowel aperture and a bag external aperture; and
a bowel connector (300; 500) adapted to connect to the inner bag (200) at the bag bowel aperture and to the bowel of the user to permit contents from the user's bowel to enter the inner bag (200) through said bowel connector and said bag bowel aperture,
wherein said contents are removable from the inner bag (200) through said bag external aperture and the distal aperture (150) of the distal member (140).

2. The device (1) according to claim **1,** wherein
the bowel connector (300; 500) adapted to connect to the inner bag (200) and the user's bowel is configured to permit contents from the bowel to enter the inner bag (200) through the bowel connector (300; 500) and the bag bowel aperture, wherein the contents are removable from the inner bag (200) through the bag external aperture; and
the device (1) further comprises a gas tunnel (142) connected to the inner bag (200) at a gas entrance inside the container (100), and a gas exit (144) through which gas may exit.

3. The device (1) of claim 1, wherein the bowel connector (300; 500) comprises an inner tube (310) and an outer tube (350) adapted to receive a portion of the bowel between the inner tube (310) and the outer tube (350).

4. The device (1) of claim 3, wherein the inner tube (310) includes one or more first balloons (312), and the outer tube (350) comprises one or more second balloons (352), wherein the first balloons (312) and the second balloons (352) are inflated to secure the portion of the bowel in place.

5. The device (1) of claim 1, wherein the bowel connector (300) comprises a plurality of inflatable balloons (312; 352) for securing the bowel in place.

6. The device (1) of claim 1, further comprising a gas tunnel (142) having a gas entrance and a gas exit (144), and a valve at the gas exit (144) adapted to release gas from within the gas tunnel (142).

7. The device (1) of claim 1, wherein the bowel connector (300) comprises one or more biocompatible surfaces.

8. The device (1) of claim 1, wherein the container (100) has a dome-shaped distal member (140).

9. The device (1) of claim 1, wherein the container (100) is made of a material being one of silicone, nylon, polypropylene, and titanium.

10. The device (1) of claim 1, wherein the bowel connector (500) comprises:
a first leg (510) adapted to connect to the inner bag (200);
a second leg (520) adapted to connect to the bowel; and
a third leg (530) adapted to connect to a second bowel downstream of the bowel.

11. The device (1) of claim 10, wherein the third leg (530) comprises a stopper (522).

12. The device (1) of claim 10, wherein the second leg (520) comprises an inner tube and an outer tube adapted to receive a portion of the bowel between the inner tube and the outer tube.

13. The device (1) of claim 10, wherein the third leg (530) comprises an inner tube and an outer tube adapted to receive a portion of the second bowel between the inner tube and the outer tube.

14. The device (1) of claim 1, wherein the inner bag (200) is made of a gas permeable material.

## Patentansprüche

1. Implantierbare Beutelvorrichtung (1) für Stoma, umfassend:
einen Behälter (100) mit einem Behälterhohlraum (160), wobei der Behälter (100) dazu konfiguriert ist, vollständig zwischen der Haut und den Bauchmuskeln eines Benutzers implantiert zu werden, wobei der Behälter (100) durch eine proximale Basis (110) mit einer Basiswand (112) und ein distales Element (140) definiert ist, wobei die proximale Basis (110) und das distale Element (140) im zusammengebauten Zustand den Behälterhohlraum (160) definieren, wobei die Basiswand (112) einen Basistunnel (122) aufweist, der eine Basisöffnung (120) definiert, die dazu bestimmt ist, mit dem Darm des Benutzers verbunden zu werden, und wobei das distale Element (140) einen distalen Tunnel (152) aufweist, der eine distale Öffnung (150) definiert, wobei der distale Tunnel (152) dazu konfiguriert ist, durch Unterhautgewebe unter der Haut des Benutzers zu verlaufen, um den Behälterhohlraum (160) mit der Außenseite der Haut zu verbinden;
eine Innentasche (200), die sich im Inneren des Behälters (100) befindet, wobei die Innentasche (200) eine Taschen-Darmöffnung und eine Taschen-Außenöffnung aufweist; und
einen Darmanschluss (300; 500), der dazu angepasst ist, mit der Innentasche (200) an der Taschen-Darmöffnung und mit dem Darm des Benutzers verbunden zu werden, um zu ermöglichen, dass der Inhalt aus dem Darm des Benutzers durch den Darmanschluss und die Taschen-Darmöffnung in die Innentasche (200) gelangt, wobei der Inhalt durch die Taschen-Außenöffnung und die distale Öffnung (150) des distalen Elements (140) aus der Innentasche (200) entnommen werden kann.

2. Vorrichtung (1) gemäß Anspruch 1, wobei der Darmanschluss (300; 500), der dazu angepasst ist, mit der Innentasche (200) und dem Darm des Benutzers verbunden zu werden, dazu konfiguriert ist, zu ermöglichen, dass der Inhalt aus dem Darm durch den Darmanschluss (300; 500) und die Taschen-Darmöffnung in die Innentasche (200) gelangt, wobei der Inhalt durch die Taschen-Außenöffnung aus der Innentasche (200) entnommen werden kann; und
wobei die Vorrichtung (1) ferner einen Gastunnel (142), der mit der Innentasche (200) an einem Gaseinlass innerhalb des Behälters (100) verbunden ist, und einen Gasauslass (144), durch den Gas austreten kann, umfasst.

3. Vorrichtung (1) nach Anspruch 1, wobei der Darmanschluss (300; 500) ein Innenrohr (310) und ein Außenrohr (350) umfasst, die dazu angepasst sind, einen Abschnitt des Darms zwischen dem Innenrohr (310) und dem Außenrohr (350) aufzunehmen.

4. Vorrichtung (1) nach Anspruch 3, wobei das Innenrohr (310) einen oder mehrere erste Ballons (312) beinhaltet und das Außenrohr (350) einen oder mehrere zweite Ballons (352) umfasst, wobei die ersten Ballons (312) und die zweiten Ballons (352) aufgeblasen werden, um den Abschnitt des Darms an Ort und Stelle zu fixieren.

5. Vorrichtung (1) nach Anspruch 1, wobei der Darmanschluss (300) eine Vielzahl von aufblasbaren Ballons (312; 352) umfasst, um den Darm an Ort und Stelle zu fixieren.

6. Vorrichtung (1) nach Anspruch 1, ferner umfassend einen Gastunnel (142) mit einem Gaseinlass und einem Gasauslass (144) und ein Ventil am Gasauslass (144), das dazu angepasst ist, Gas aus dem Inneren des Gastunnels (142) abzulassen.

7. Vorrichtung (1) nach Anspruch 1, wobei der Darmanschluss (300) eine oder mehrere biokompatible Oberflächen umfasst.

8. Vorrichtung (1) nach Anspruch 1, wobei der Behälter (100) ein kuppelförmiges distales Element (140) aufweist.

9. Vorrichtung (1) nach Anspruch 1, wobei der Behälter (100) aus einem Material hergestellt ist, das eines von Silikon, Nylon, Polypropylen und Titan ist.

10. Vorrichtung (1) nach Anspruch 1, wobei der Darmanschluss (500) Folgendes umfasst:
einen ersten Schenkel (510), der dazu angepasst ist, mit der Innentasche (200) verbunden zu werden;
einen zweiten Schenkel (520), der dazu angepasst ist, mit dem Darm verbunden zu werden; und
einen dritten Schenkel (530), der dazu angepasst ist, mit einem zweiten Darm stromabwärts des Darms verbunden zu werden.

11. Vorrichtung (1) nach Anspruch 10, wobei der dritte Schenkel (530) einen Stopfen (522) umfasst.

12. Vorrichtung (1) nach Anspruch 10, wobei der zweite Schenkel (520) ein Innenrohr und ein Außenrohr umfasst, die dazu angepasst sind, einen Abschnitt des Darms zwischen dem Innenrohr und dem Außenrohr aufzunehmen.

13. Vorrichtung (1) nach Anspruch 10, wobei der dritte Schenkel (530) ein Innenrohr und ein Außenrohr umfasst, die dazu angepasst sind, einen Abschnitt des zweiten Darms zwischen dem Innenrohr und dem Außenrohr aufzunehmen.

14. Vorrichtung (1) nach Anspruch 1, wobei die Innentasche (200) aus einem gasdurchlässigen Material hergestellt ist.

## Revendications

1. Dispositif poche d'ostomie implantable (1) comprenant :
un récipient (100) comportant une cavité de récipient (160), le récipient (100) étant conçu pour être entièrement implanté entre la peau et les muscles abdominaux d'un utilisateur, le récipient (100) étant défini par une base proximale (110) dotée d'une paroi de base (112) et un élément distal (140), la base proximale (110) et l'élément distal (140) définissant la cavité de récipient (160) lorsqu'ils sont assemblés, la paroi de base (112) comportant un tunnel de base (122) définissant une ouverture de base (120) conçue pour être raccordée à l'intestin de l'utilisateur, et l'élément distal (140) comportant un tunnel distal (152) définissant une ouverture distale (150), le tunnel distal (152) étant conçu pour traverser les tissus sous-cutanés sous la peau de l'utilisateur, de manière à raccorder la cavité de récipient (160) à l'extérieur de la peau ;
un sac interne (200) situé à l'intérieur du récipient (100), le sac interne (200) comportant une ouverture de sac pour intestin et une ouverture de sac externe ; et
un raccord d'intestin (300 ; 500) adapté au raccordement au sac interne (200) au niveau de l'ouverture de sac pour intestin et à l'intestin de l'utilisateur de manière à permettre au contenu de l'intestin de l'utilisateur d'entrer dans le sac interne (200) par ledit raccord d'intestin et ladite ouverture de sac pour intestin,
ledit contenu pouvant être retiré du sac interne (200) par ladite ouverture de sac externe et l'ouverture distale (150) de l'élément distal (140).

2. Dispositif (1) selon la revendication 1,
ledit raccord d'intestin (300 ; 500) adapté au raccordement au sac interne (200) et à l'intestin de l'utilisateur étant conçu pour permettre au contenu de l'intestin d'entrer dans le sac interne (200) par le raccord d'intestin (300 ; 500) et l'ouverture de sac pour intestin, ledit contenu pouvant être retiré du sac interne (200) par l'ouverture externe de la poche ; et
ledit dispositif (1) comprenant en outre un tunnel de gaz (142) raccordé au sac interne (200) au niveau d'une entrée de gaz à l'intérieur du récipient (100), et une sortie de gaz (144) à travers laquelle le gaz peut sortir.

3. Dispositif (1) selon la revendication 1, ledit raccord d'intestin (300 ; 500) comprenant un tube interne (310) et un tube externe (350) adaptés à la réception d'une partie de l'intestin entre le tube interne (310) et le tube externe (350).

4. Dispositif (1) selon la revendication 3, ledit tube interne (310) comprenant un ou plusieurs premiers ballonnets (312), et ledit tube externe (350) comprenant un ou plusieurs seconds ballonnets (352), lesdits premiers ballonnets (312) et lesdits seconds ballonnets (352) étant gonflés de manière à fixer la partie de l'intestin en place.

5. Dispositif (1) selon la revendication 1, ledit raccord d'intestin (300) comprenant une pluralité de ballonnets gonflables (312 ; 352) destinés à fixer l'intestin en place.

6. Dispositif (1) selon la revendication 1, comprenant en outre un tunnel de gaz (142) comportant une entrée de gaz et une sortie de gaz (144), et une vanne au niveau de la sortie de gaz (144) adaptée à la libération du gaz de l'intérieur du tunnel de gaz (142).

7. Dispositif (1) selon la revendication 1, ledit raccord d'intestin (300) comprenant une ou plusieurs surfaces biocompatibles.

8. Dispositif (1) selon la revendication 1, ledit récipient (100) comportant un élément distal en forme de dôme (140).

9. Dispositif (1) selon la revendication 1, ledit récipient (100) étant constitué d'un matériau parmi le silicone, le nylon, le polypropylène et le titane.

10. Dispositif (1) selon la revendication 1, ledit raccord d'intestin (500) comprenant :
une première branche (510) adaptée au raccordement au sac interne (200) ;
une deuxième branche (520) adaptée au raccordement à l'intestin ; et
une troisième branche (530) adaptée au raccordement à un second intestin en aval de l'intestin.

11. Dispositif (1) selon la revendication 10, ladite troisième branche (530) comprenant une butée (522).

12. Dispositif (1) selon la revendication 10, ladite deuxième branche (520) comprenant un tube interne et un tube externe adaptés à la réception d'une partie de l'intestin entre le tube interne et le tube externe.

13. Dispositif (1) selon la revendication 10, ladite troisième branche (530) comprenant un tube interne et un tube externe adaptés à la réception d'une partie du second intestin entre le tube interne et le tube externe.

14. Dispositif (1) selon la revendication 1, ledit sac interne (200) étant constitué d'un matériau perméable aux gaz.
